(19) Europäisches Patentamt
European Patent Office
Office européen des brevets

(11) **EP 3 219 251 A1**

(12) **EUROPEAN PATENT APPLICATION**
published in accordance with Art. 153(4) EPC

(43) Date of publication:
**20.09.2017 Bulletin 2017/38**

(51) Int Cl.:
**A61B 5/00** (2006.01)  **A61B 5/107** (2006.01)
**H04N 5/225** (2006.01)

(21) Application number: **15858899.6**

(22) Date of filing: **15.10.2015**

(86) International application number:
**PCT/JP2015/079123**

(87) International publication number:
**WO 2016/076059 (19.05.2016 Gazette 2016/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **12.11.2014 JP 2014230034**

(71) Applicant: **Konica Minolta, Inc.
Tokyo 100-7015 (JP)**

(72) Inventor: **MATSUDA, Shinya
Tokyo 100-7015 (JP)**

(74) Representative: **Alton, Andrew
Urquhart-Dykes & Lord LLP
Arena Point
Merrion Way
Leeds LS2 8PA (GB)**

(54) **ORGAN IMAGE CAPTURE DEVICE AND PROGRAM**

(57)    An organ image capture device (1) is provided with an imaging unit (3), a display unit (4), and an information extracting unit (14). The imaging unit (3) images an organ of a living body and acquires an image thereof. The display unit (4) displays, together with the image acquired by imaging by the imaging unit (3), an indicator for specifying an organ imaging position satisfying an imaging condition including a condition relating to illumination at the time of imaging by the imaging unit (3). The information extracting unit (14) extracts information necessary for diagnosing the degree of health of the living body from an image which is acquired by imaging by the imaging unit (3) and is within a range specified by the indicator.

FIG.2

## Description

### Technical Field

The present invention relates to an organ image capture device for imaging an organ of a living body to extract information needed for diagnosis of healthiness, and a program for causing a computer to execute each processing of the organ image capture device.

### Background Art

Methods of diagnosis using living-body images are widely used in the field of Western medicine. Based on such methods, not only the skin, which is the surface of human body parts such as the face, hands, and legs, but also membrane tissues of the lips and the eyelids are diagnosed, and further, the digestive organs, such as the stomach and the intestines, and other internal body organs are diagnosed by using X rays, supersonic waves, or the like.

In Oriental medicine, on the other hand, methods of diagnosis (visual diagnosis, tongue diagnosis) are known in which the conditions of the human face and the human tongue are examined to diagnose health condition and disease condition. Examples of quantities of features necessary in the tongue diagnosis include the color and the sharpness of an imaging object (the tongue, the tongue coating), irregularities and the degree of gloss (moisture) on the surface of the imaging object, etc. Devices for supporting the diagnosis of healthiness by imaging the tongue are required to have functions of correctly reproducing colors in imaging the tongue and reading detailed information (image resolution), for example, and they are further required to have a function of appropriately reproducing irregularities and the degree of gloss on the surface of the tongue. Here, the color temperature of illumination light, the number of pixels of an imaging sensor, the lens resolution, and the illumination angle at which the illumination light is shone to the imaging object also need to be considered as important factors of the imaging conditions.

Patent Documents 1 to 3 listed below, for example, propose techniques for appropriately setting the imaging conditions. Patent Document 1 discloses a technique for adjusting the amount of illumination light according to the distance from the imaging object in close-up imaging. Patent Document 2 discloses a technique for arranging an illuminator having an arm in the lens barrel to arbitrarily set the irradiation angle. Patent Document 3 discloses a technique for controlling the focal distance according to the illumination angle. Although not directed to the optimization of the imaging conditions, patent Documents 4 to 7 suggest techniques for facilitating imaging by displaying a template for reference of the composition.

### Citation List

### Patent Documents

Patent Document 1: Japanese Patent Application Publication No. 2005-152569 (claim 1, paragraphs [0018], [0019], FIG. 2, etc.)
Patent Document 2: Japanese Patent Application Publication No. 2002-229106 (claim 1, paragraphs [0020], [0021], FIG. 1, etc.)
Patent Document 3: Japanese Patent Application Publication No. H07-5530 (claim 1, paragraphs [0011], [0012], FIG. 1, FIG. 2, etc.)
Patent Document 4: Japanese Patent Application Publication No. 2007-166250 (claim 1, FIG. 3, etc.)
Patent Document 5: Japanese Patent Application Publication No. 2011-135527 (claim 1, FIG. 5, etc.)
Patent Document 6: Japanese Patent Application Publication No. 2009-33544 (claims 1 and 2, FIG. 2, etc.)
Patent Document 7: Japanese Patent Application Publication No. 2004-40724 (claim 1, FIG. 4, etc.)

### Summary of Invention

### Technical Problem

However, with the techniques disclosed in Patent Documents 1 to 3, the illumination angle, which is one of conditions regarding illumination and is important in the imaging of a living body, needs to be set manually. Professional photographers and experienced engineers are able to appropriately set the illumination angle, but it requires know-how to appropriately set the illumination angle, and thus is difficult for beginners. What is more, even if a beginner is able to appropriately set a variety of imaging conditions including the illumination angle, it is difficult for him or her to set the imaging conditions to be the same for imaging on different dates or of different examinees. Thus, there is a problem that it is impossible to easily and stably obtain an optimum image for the diagnosis of healthiness.

There sure is a method in which a dedicated imaging apparatus with fixed imaging conditions is prepared, but such a method is disadvantageous in terms of size and cost, and further, has low freedom with respect to locations for imaging. As mentioned above, Patent Documents 4 to 7 disclose nothing regarding the optimization of imaging conditions.

The present invention has been made to solve the above problems, and an object of the present invention is to provide an organ image capture device with which even an inexperienced user can easily and stably obtain an image most appropriate for diagnosis of the degree of healthiness, and a program for causing a computer to execute each processing of the organ image capture device.

## Solution to Problem

An organ image capture device according to one aspect of the present invention includes an imager which images an organ of a living body to acquire an image, a display which displays, along with the image acquired through imaging performed by the imager, an index for specifying an imaging position of the organ where the organ is to be located when the imager images the organ, the imaging position satisfying imaging conditions including a condition regarding illumination, and an information extractor which extracts information necessary for diagnosing a degree of healthiness of the living body, from an image which is acquired through imaging performed by the imager and which is located inside a range specified by the index.

A program according to another aspect of the present invention is a program for causing a computer to execute processing of displaying, on a display, along with an image acquired through imaging performed by the imager, an index for specifying an imaging position of an organ of a living body where the organ is to be located when an imager images the organ, the imaging position of the organ satisfying imaging conditions including a condition regarding illumination, and processing of extracting information necessary for diagnosing a degree of healthiness of the living body from an image which is acquired through imaging performed by the imager and which is located inside a range specified by the index.

## Advantageous Effects of Invention

With the features described above, even an inexperienced user is able to use the device to easily and stably acquire an image most appropriate for diagnosis of the degree of healthiness, on any day, with respect to any examinee.

## Brief Description of the Drawings

FIG. 1 is a perspective view illustrating an exterior appearance of an organ image capture device according to one embodiment of the present invention;
FIG. 2 is a block diagram illustrating an outline of a configuration of the organ image capture device;
FIG. 3 is an explanatory diagram illustrating a positional relationship of an illuminator and an imager with respect to an imaging object;
FIG. 4 is an explanatory diagram illustrating an example of a captured image of a tongue, an example of an edge extraction filter, and a contour line of the tongue extracted by using the edge extraction filter;
FIG. 5 is an explanatory diagram illustrating typical diagnosis items regarding the tongue and tongue coating;
FIG. 6 is an explanatory diagram illustrating an example of a frame line displayed on a display;
FIG. 7 is an explanatory diagram illustrating another example of the frame line;
FIG. 8 is an explanatory diagram illustrating an example of a position specification line displayed on the display;
FIG. 9 is an explanatory diagram illustrating another example of the position specification line;
FIG. 10 is an explanatory diagram illustrating a captured image of the tongue along with diagnostic regions;
FIG. 11 is an explanatory diagram illustrating a horizontal distribution of RGB image data of the captured image of the tongue;
FIG. 12 is an explanatory diagram illustrating the captured image of the tongue along with its sectional shapes;
FIG. 13 is an explanatory diagram illustrating the contour line of the tongue and a detection region for detecting moisture on the tongue;
FIG. 14 is a graph illustrating a spectrum distribution on the tongue;
FIG. 15 is a graph schematically illustrating a frequency distribution of B image data extracted from the detection regions;
FIG. 16 is a graph illustrating the contour line of the tongue and an approximate curve of the contour line;

FIG. 17 is a graph plotting difference in y-coordinate between the contour line and the approximate curve;

FIG. 18 is an explanatory diagram illustrating a tongue having cracks in its surface;

FIG. 19 is a graph schematically illustrating the frequency distribution of B image data in a case of a tongue having no crack in its surface and in a case of a tongue having some racks in its surface;

FIG. 20 is a flow chart illustrating a flow of operation in the organ image capture device; and

FIG. 21 is an explanatory diagram illustrating a captured image of a human lower eyelid along with a frame line.

**Description of Embodiments**

An embodiment of the present invention will be described below with reference to the accompanying drawings. In this description, when a numerical value range is indicated as A to B, the lower limit A and the upper limit A are both included in the numerical value range.

[Outline of Configuration of Organ Image Capture Device]

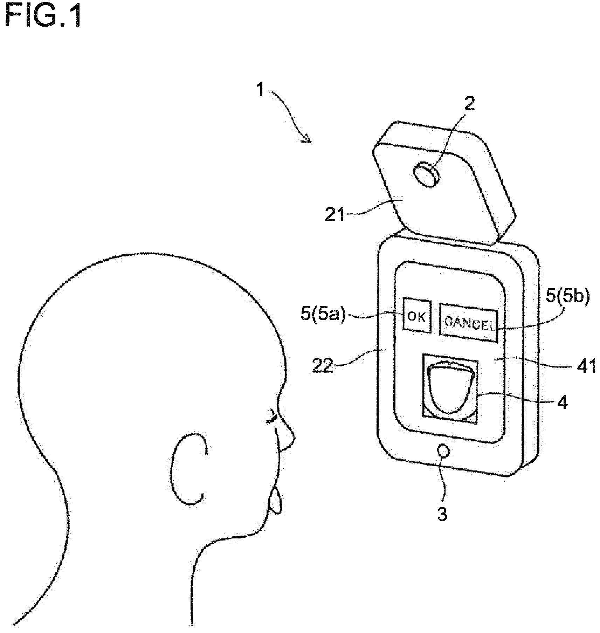

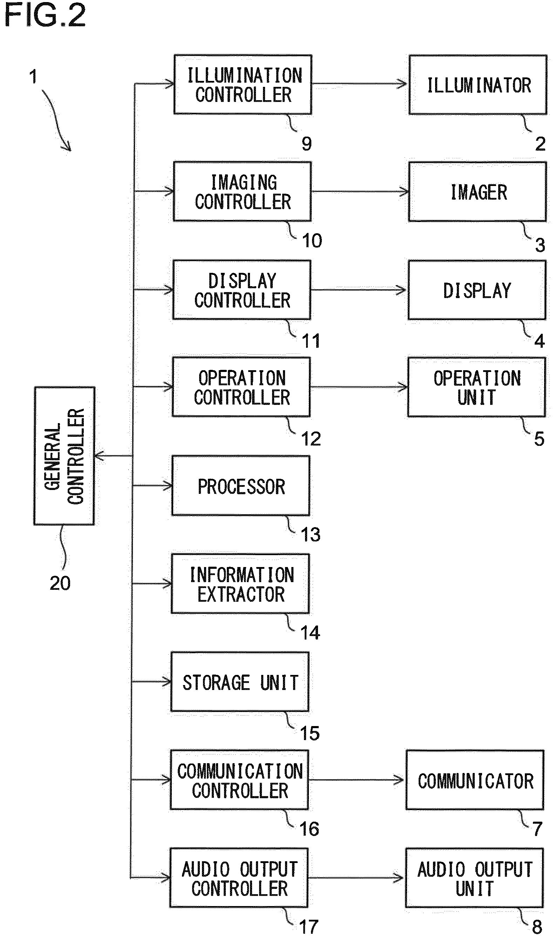

FIG. 1 is a perspective view illustrating an exterior appearance of an organ image capture device 1 according to the present embodiment, and FIG. 2 is a block diagram illustrating an outline of a configuration of the organ image capture device 1. The organ image capture device 1 includes an illuminator 2, an imager 3, a display 4, an operation unit 5, a communicator 7, and an audio output unit 8. The illuminator 2 is provided in a housing 21, and the imager 3, the display 4, the operation unit 5, the communicator 7, and the audio output unit 8 are provided in a housing 22. The housing 21 is provided rotatable with respect to the housing 22 so that an illumination angle of the illuminator 2 can be changed.

(Illuminator)

The illuminator 2 illuminates an organ (here, the tongue) of a living body as an imaging object, and is configured as a light that illuminates the imaging object from above. The illuminator 2 includes a light source that emits light of a daylight color, such as a xenon lamp, for improved color rendering. The brightness of the light source is controlled by an illumination controller 9, in accordance with the sensitivity of the imager 3 and the distance to the imaging object. For example, the brightness of the light source is so controlled as to provide an illuminance of 1000 to 10000 lx on the imaging object. The illuminator 2 includes, in addition to the light source, a lighting circuit and a dimming circuit, and is controlled according to instructions from the illumination controller 9 so as to be lit, extinguished, and dimmed.

(Imager)

The imager 3 images an organ (such as the tongue) of a living body to acquire an image of it under the illumination provided by the illuminator 2. The imager 3 includes an imaging lens and an area sensor (an image sensor). In the imaging lens, the aperture (the fastness of the lens), the shutter speed, and the focal length are set such that the entire imaging object is in focus. For example, the f-number may be set to 16, the shutter speed may be set to 1/120 seconds, and the focal length may be set to 20 mm.

The area sensor includes, for example, an image sensor such as a CCD (charge-coupled device) image sensor or a CMOS (complementary metal oxide semiconductor) sensor, and its sensitivity, resolution, and the like are so set that the color and the shape of the imaging object can be detected in a satisfactory manner. For example, its sensitivity may be set to 60 db, and its resolution may be set to 10 megapixels.

The imaging performed by the imager 3 is controlled by an imaging controller 10. The imager 3 further includes, in addition to the imaging lens and the area sensor, a focusing mechanism, an aperture mechanism, a drive circuit, an A/D conversion circuit, and the like, none of which is illustrated, and is controlled according to instructions from the imaging controller 10 in terms of focus, aperture, A/D conversion, and the like. The imager 3 acquires, as the data of a captured image, data of, for example, eight bits, representing a value from 0 to 255, for each of red (R), green (G), and blue (B) per pixel. That is, the imager 3 acquires a captured image of an imaging object of each of the three different colors (RGB), and thereby image data of each color is acquired. The power of an imaging lens of the imager 3 is so controlled by the imaging controller 10 that a required image resolution can be obtained.

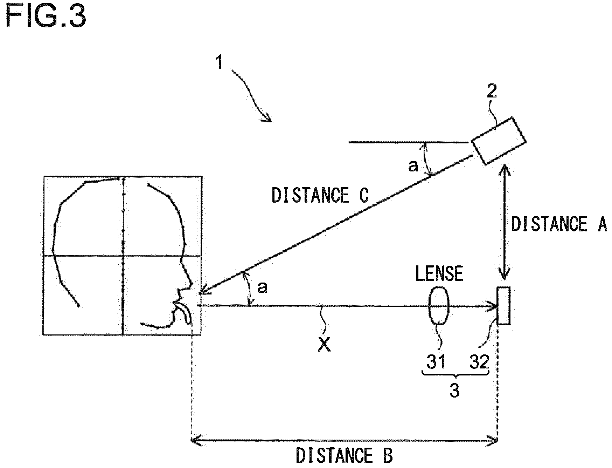

FIG. 3 is an explanatory diagram illustrating a positional relationship of the illuminator 2 and the imager 3 with respect to an imaging object (the tongue or the face). As illustrated in the figure, the imager 3, which has at least an imaging lens 31 and an area sensor 32, is arranged right in front of the imaging object. The illuminator 2 is so arranged as to illuminate the imaging object at an illumination angle "a" of 0° to 45°, for example, with respect to an imaging optical axis X of the imager 3, which passes through the imaging object. The imaging optical axis X denotes the optical axis of the imaging lens provided in the imager 3.

With illumination applied at a large illumination angle "a", surface irregularities can be measured with improved accuracy, but the shadow of the upper lip reduces the imageable area over which the tongue can be imaged. Conversely, with illumination applied at a small illumination angle "a", the imageable area is increased, but the accuracy of measurement is degraded and specular reflection of illumination light causes severe color clipping. Out of these considerations, a preferred range of the illumination angle "a" is from 15° to 30°. In particular, in a case of examining an organ of a living body, such as the tongue, the illumination angle "a" is preferably about 30°. In the present embodiment, in view of the detection of moisture and a tooth mark on the tongue, details of which will be described later, too, the illumination angle "a" at which illumination is applied to the organ by the illuminator 2 is set to 30°.

(Display)

The display 4 includes a liquid crystal panel, a backlight, a lighting circuit, and a control circuit, of which none is illustrated, and displays an image acquired through imaging performed by the imager 3 and a frame line. As the image displayed on the display 4, in addition to an image acquired through final imaging performed by the imager 3, an image (a preview image) acquired through preliminary imaging, which is performed before final imaging, is also included. The frame line mentioned above is a picture frame for specifying an imaging position of an organ (here, the tongue) where the organ is to be located when the imager 3 images the organ, and details of the frame line will be described later.

The display 4 can also display information (e.g., the result of a diagnosis made by an external medical facility based on information transmitted to it) acquired from outside via the communicator 7. The display of a variety of information on the display 4 is controlled by a display controller 11.

Here, the image of the imaging object acquired through imaging performed by the imager 3 may be displayed on the display 4 after subjected to image processing, such as thinning-out processing and color correction processing, performed by an image processing unit (not shown). In the following description, unless otherwise specified, when an image is simply described as "(captured) image of the imaging object", it denotes an image which has been acquired through imaging performed by the imager 3 but has not yet been subjected to the above-described image processing necessary for display.

(Operation Unit)

The operation unit 5 is an input unit via which to instruct the imager 3 to perform imaging, and includes an OK button (TAKE IMAGE button) 5a and a CANCEL button 5b. In the present embodiment, the display 4 and the operation unit 5 are constituted by a single touch panel display device 41 (see FIG. 1). The display of the operation unit 5 on the touch panel display device 41 is controlled by an operation controller 12. Here, the operation unit 5 may be configured as any other input device than the touch panel display device 41 (the operation unit 5 may be provided anywhere else than inside the display area of the touch panel display device 41).

(Communicator)

The communicator 7 is an interface via which to transmit information obtained by a later-described information extractor 14 to an external device, and conversely, to receive a variety of information (e.g., the result of a diagnosis made by an external medical facility) transmitted from an external device. The transmission and reception of information by the communicator 7 is controlled by a communication controller 16.

(Audio Output Unit)

The audio output unit 8 outputs a variety of information in the form of sound, and is constituted by a speaker, for example. The audio output unit 8 can output, in the form of sound, the result of a diagnosis made based on information extracted by the information extractor 14. The result of a diagnosis here may be one obtained by the present device, or may be the result of a diagnosis made by an external facility transmitted to the present device. The output of sound from the audio output unit 8 is controlled by an audio output controller 17.

(Controller and Others)

The organ image capture device 1 includes the illumination controller 9, the imaging controller 10, the display controller 11, the operation controller 12, a processor 13, the information extractor 14, a storage unit 15, the communication controller 16, the audio output controller 17, and a general controller 20, which controls all these blocks. As described above, the illumination controller 9, the imaging controller 10, the display controller 11, the operation controller 12, the communication controller 16, and the audio output controller 17 control the illuminator 2, the imager 3, the display 4, the operation unit 5, the communicator 7, and the audio output unit 8, respectively. The general controller 20 is constituted by a central processing unit (CPU), for example, and executes an operation program stored in the storage unit 15. Here, the illumination controller 9, the imaging controller 10, the display controller 11, the operation controller 12, the communication controller 16, the audio output controller 17, and the general controller 20 may be integrally constituted (for example, by a single CPU).

(Processor)

The processor 13 is an image processing unit which performs processing of extracting a contour line of an organ from an image acquired by the imager 3. In the present embodiment, the processor 13 extracts a contour line of the tongue as an organ, based on a luminance edge (a part of the image where brightness changes sharply) in the captured image.

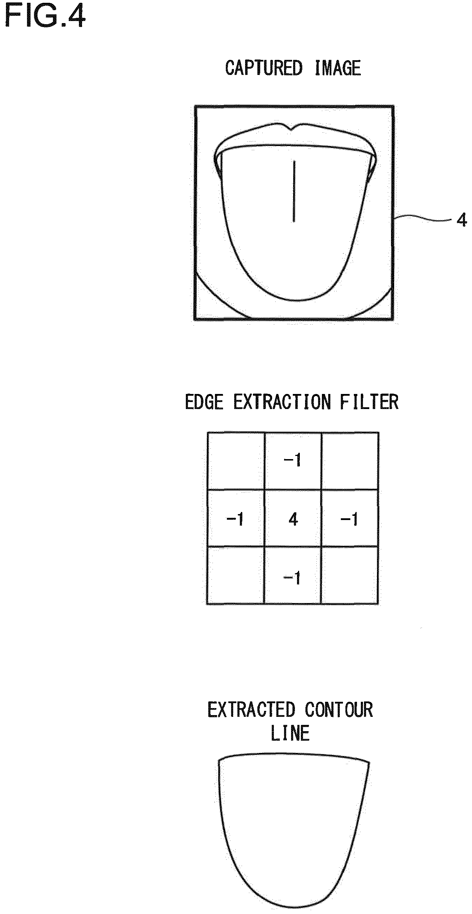

The extraction of the luminance edge can be performed, for example, by using an edge extraction filter as shown in FIG. 4. The edge extraction filter is a filter that gives weights to pixels near a pixel of interest in performing first-order differentiation (in calculating differences in image data between neighboring pixels). By using such an edge extraction filter, for example, with respect to the G image data of each pixel in a captured image, differences in image data are calculated between the pixel of interest and neighboring pixels, and such pixels as yield differences exceeding a predetermined threshold value are extracted; in this way, pixels that constitute a luminance edge can be extracted. Around the tongue, its shadow produces luminance differences; thus, by extracting pixels that constitute a luminance edge in the manner described above, it is possible to extract a contour line of the tongue. Here, G image data is used in the calculation because it has the greatest influence on luminance; however, red or blue image data may be used instead.

(Storage Unit)

The storage unit 15 is a memory, and stores therein data of an image acquired by the imager 3, information acquired by the processor 13 and the information extractor 14, information received from an external device, and the like, and also stores therein programs for operating the various controllers described above. Examples of such a memory include a RAM, a ROM, a nonvolatile memory, and the like.

(Information Extractor)

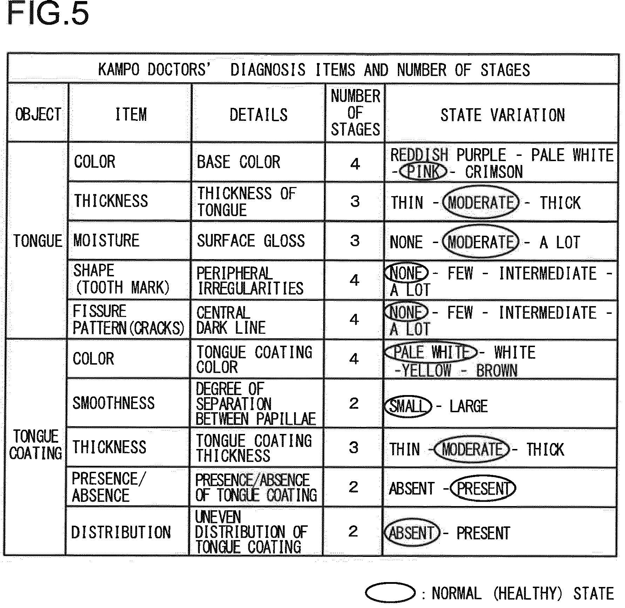

The information extractor 14 is a processor which extracts information necessary for diagnosis, from an image of an imaging object acquired through imaging performed by the imager 3. Here, FIG. 5 illustrates typical diagnosis items regarding the tongue and tongue coating, used in Oriental medicine. As for the tongue, five items are commonly used as the diagnosis items, namely, the color of the tongue, the thickness of the tongue, the moisture (the degree of gloss) on the surface of the tongue, the shape of the tongue (a tooth mark on the tongue), and a fissure pattern (cracks in the surface of the tongue). As for the tongue coating, five items are used as the diagnosis items, namely, the color of the tongue coating, the smoothness of the tongue coating (the degree of separation between papillae), the thickness of the tongue coating, the presence/absence of the tongue coating, the state of distribution of the tongue coating. In the column of the item "state variation" in FIG. 5, the circle in each cell indicates the normal (healthy) state, and terms away from the circled terms indicating normal states indicate abnormal (sick) states.

In a case where the imaging object is the tongue of a living body, according to the present embodiment, the information extractor 14 extracts, from an image of the imaging object captured through final imaging, information necessary for diagnosis, such as information regarding the moisture on the tongue, a tooth mark on the tongue, the color of the tongue, the thickness of the tongue, a fissure pattern in the tongue, and the smoothness of the tongue coating. A specific method of extracting the variety of information will be described later.

[Relationship between Diagnosis Items and Imaging Conditions]

Here, a description will be given of the relationship between the above-mentioned diagnosis items and imaging conditions including illumination conditions.

(Relationships of Moisture and Tooth Mark on Tongue with Imaging Conditions)

The detection of moisture on a tongue and the detection of a tooth mark on the tongue require an illumination-angle condition, a condition regarding the illumination angle, of the illuminator 2. As will be described later, the degree of moisture on the tongue is determined depending on the degree of gloss on the tongue detected by measuring components of illumination light reflected on the surface of the tongue. When the degree of moisture is high, the degree of gloss on the surface of the tongue is also high, and the amount of reflected components of illumination light is increased. Conversely, when the degree of moisture is low, the degree of gloss on the surface of the tongue is also low, and the amount of reflected components of illumination light is reduced. By setting the illumination angle to about 30° as described above, it is possible to appropriately detect reflected components of the illumination light from a curved upper area of the tongue, and thereby, it is possible to appropriately detect the degree of moisture on the tongue.

As for the severity of a tooth mark, the contour line of the tongue is detected, and the severity of a tooth mark is determined according to the degree of irregularities in the contour line of the tongue. The face, the lips, and the tongue are all organs of the same living body, it is difficult to detect the contour line of the tongue depending only on color differences. To address this inconvenience, shadow (shade, dark area) is made around the tongue by means of the illumination light, and by detecting the shadow, it becomes easy to extract the contour line of the tongue. Here, too, by setting the illumination angle to about 30°, appropriate shadings can be produced around the tongue, and thereby, the contour line of the tongue can be detected appropriately.

(Relationships of Fissure Pattern on Tongue and Smoothness of Coating with Imaging Conditions)

The detection of a fissure pattern in the tongue and the detection of the smoothness of the tongue coating require an imaging-resolution condition, a condition regarding imaging resolution. The presence/absence of a tongue fissure pattern and the smoothness of the tongue coating are each judged by detecting the state of papillae existing on the surface of the tongue. A fissure pattern in the tongue is caused by partial lack or partially incomplete growth of papillae. Further, if layers of old tissue are formed one on another due to incomplete papillae turnover, the surface of the tongue coating loses graininess and becomes smooth (unfavorable state).

Each papilla is about 0.5 mm in diameter. The width and the length of the tongue is about 50 mm on average. To separate the papillae from each other and appropriately detect the presence and absence of a fissure pattern in the tongue and the smoothness of the tongue coating, the resolution of at least $200 \times 200 = 40000$ pixels, desirably of $400 \times 400 = 160000$ pixels, is necessary.

(Relationships of Color and Thickness of Tongue with Imaging Conditions)

The detection of the color of the tongue and the thickness of the tongue require an illuminance condition, a condition regarding illuminance provided on the tongue when the illuminator 2 illuminates the tongue. With a low illuminance, the S/N ratio of an imaging signal (image data of a captured image) is reduced, which makes correct detection and diagnosis difficult. In a case where illumination light comes also from a fluorescent lamp, for example, existing in the imaging environment, influence of factors such as the chromaticity, the flicker, and the angle of the fluorescent lamp also needs to be considered. The illuminance in an in-door environment is typically about 100 to 500 lx, and to eliminate its influence, it is necessary for the illuminator 2 to provide illumination of an illuminance several times to ten and several times as high as the illuminance of the illumination provided by the fluorescent lamp (that is, for example, about 1000 to 10000 lx).

[Specific Method for Setting Imaging Position satisfying Imaging Conditions]

Next, a description will be given of a specific method for setting an imaging position that satisfies the imaging conditions.

(Imaging Distance)

First, in FIG. 3, an imaging distance B required is calculated from a distance A between the imaging optical axis X and the illuminator 2 and the illumination angle "a" most appropriate to the organ. For example, in a case where the distance A is 100 mm, and the illumination angle "a" is 30°, the imaging distance B is appropriately about 170 mm ($B = A/\tan 30° = 100/(1/\sqrt{3}) \approx 170$).

(Imaging Magnification)

In a case where the pixel pitch of an imaging sensor of the imager 3 is 10 $\mu$m, the 400 pixels, which need to be arranged on each side to extract information necessary for diagnosis from a captured image is equivalent to 4 mm (10 $\mu$m $\times$ 400 = 4000 $\mu$m). The tongue of the average size can fit in a rectangular region each side of which is 50 mm in length, and thus, the imaging magnification will be $\times 0.08$ (4 mm $\div$ 50 mm = 0.08).

(Brightness of Light Source)

In the above-described case where the distance A is 100 mm and the illumination angle "a" is 30°, a distance C, which is a distance from the illuminator 2 to the imaging object, becomes 200 mm (C=A/sin 30° = 100/(1/2) = 200). In a case where the illuminator 2 illuminates an area of 100 mm in every direction, which is twice as large as the tongue, and is placed at a position away from the tongue by the distance C (200 mm), in order to obtain a required illuminance on the imaging object (for example, 5000 lx), the illuminator 2 needs to have a light source of 50 lm (5000 lx × 0.1 m × 0.1 m = 50 lm).

Hence, the imaging distance B, the imaging magnification, and the brightness of the light source are determined depending on the required imaging conditions (illuminance, illumination angle, resolution). The imaging magnification and the brightness can be adjusted to their required values by controllers (the illumination controller 9, the imaging controller 10) respectively controlling the illuminator 2 and the imager 3. The imaging distance B can be set by displaying, on the display 4, a frame line indicating a position of the tongue at the time of imaging performed at the imaging distance B. That is, looking at the frame line displayed on the display 4, the user adjusts the imaging position of the tongue such that a captured image (a preview image) of the tongue is located within the frame line, and thereby the imaging distance B can be set. A position located at the imaging distance B satisfies the required imaging conditions (required illuminance, illumination angle, and resolution) described above, and thus by imaging the tongue at the position, it is possible to acquire the most appropriate image from which to detect information regarding the color of the tongue and the like.

[Example of Display of Frame Line]

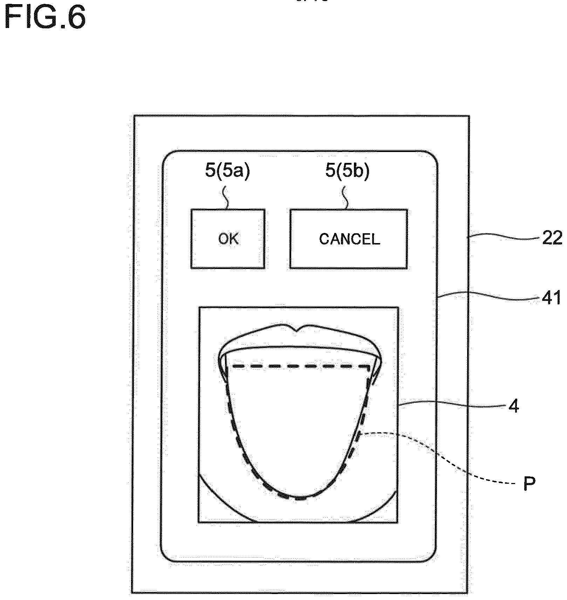

FIG. 6 illustrates an example of a frame line P displayed on the display 4. As described above, the frame line P is a frame-shaped line provided to specify an imaging position on the display, the imaging position satisfying the imaging conditions. The imaging conditions include conditions (illumination conditions) regarding illumination. The illumination conditions include at least either the illumination-angle condition regarding the illumination angle of the illuminator 2 at the time of imaging or the illuminance condition regarding the illuminance on the tongue at the time of imaging. Accordingly, satisfaction of the imaging conditions includes at least satisfaction of the illumination-angle condition or satisfaction of the illuminance condition. Here, satisfaction of the illumination-angle condition means that an angle required as the illumination angle (for example, an illumination angle of 30° with respect to the imaging optical axis) is obtained. Satisfaction of the illuminance condition means that a value (for example 5000 lx) required as the illuminance on the tongue is obtained.

Further, the imaging conditions may include, in addition to the illumination conditions described above, an image-resolution condition, which is a condition regarding the resolution of an image captured by the imager 3. In this case, satisfaction of the imaging conditions includes satisfaction of the image-resolution condition, and this means that a required resolution (400 × 400 pixels, for example) can be obtained as the resolution of the captured image.

The frame line P is displayed in a center of the display 4, and drawn, in the similitude of the ideal tongue shape, with a line (short dashed line) upper parts of which extend to be increasingly more away from each other in the left-right direction with respect to the display 4, and a lower part of the line (short dashed line) is curved (semi-ellipse).

The display 4 displays in real time a video image of the tongue obtained in the preliminary imaging. The user can place the tongue within the frame line P on the display 4 by changing the position of the tongue by moving it in any of forward, backward, leftward, rightward, upward, and downward directions relative to the imager 3, and by adjusting an angle of the tongue (how the tongue is stuck out) with respect to the imaging optical axis X of the imager 3.

The height/width ratio of the displayed frame line P is appropriately in the range of height : width = 1 : 0.5 to 1.5. Various researches have shown that when the tongue is sufficiently stuck out of the mouth in a relaxed manner, the proportion of height : width of the tongue is within the range of approximately height: width = 1 : 0.7 to 1.1. Hence, when the height/width ratio of the frame line P is set to be in the range of 0.5 to 1.5, desirably in the range of height : width = 1 : 0.7 to 1.1, more desirably around height : width = 1 : 0.9, almost the whole tongue can be located within the frame line P, and this makes it possible to perform imaging (final imaging) of the tongue in an appropriate state.

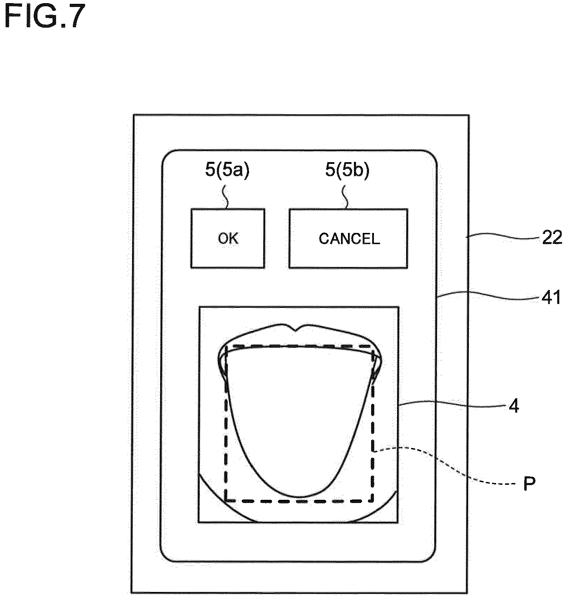

FIG. 7 illustrates another example of the frame line P displayed on the display 4. As illustrated in the figure, the frame line P may be a line forming a rectangle, defining top, bottom, left, and right sides of the rectangle. Further, there is no particular limitation to the type of the line with which to draw the frame line P, and it may be drawn with a short dashed line as in FIG. 6 and FIG. 7, or, although not illustrated, it may be drawn with a solid line.

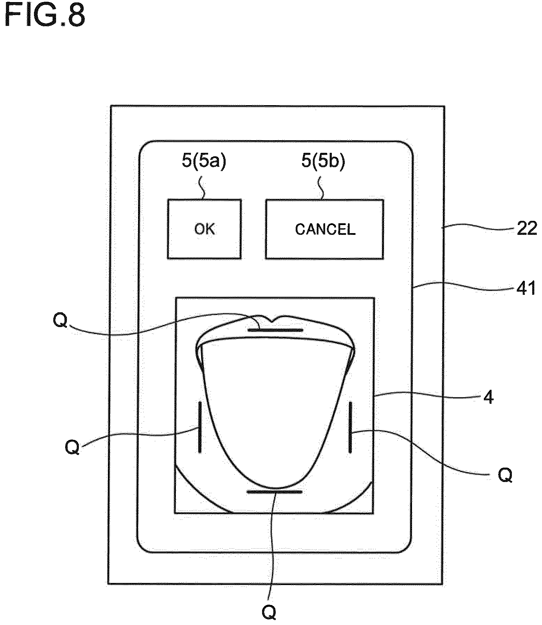

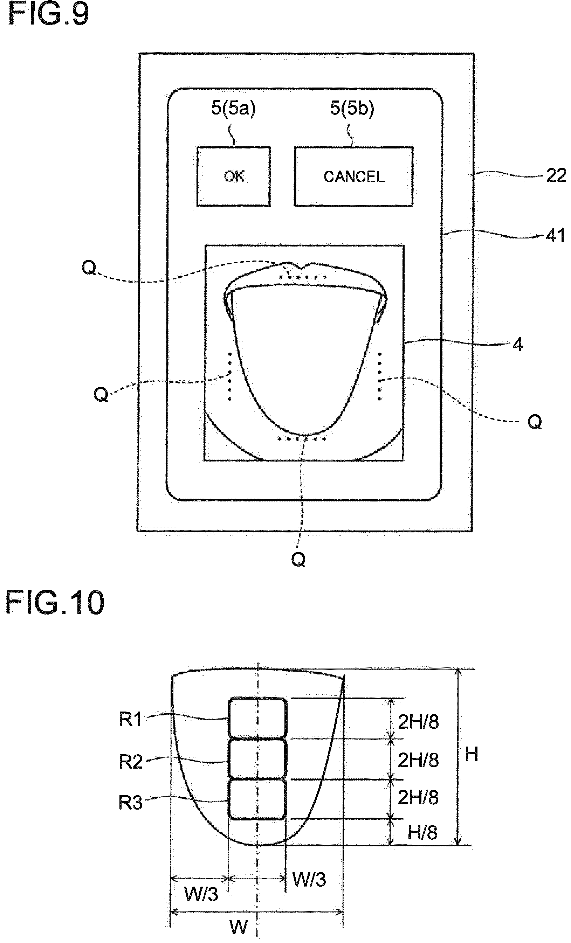

In the above description, the frame line P has been dealt with as an index specifying, on the display screen of the display 4, the imaging position that satisfies the imaging conditions, but the index does not necessarily need to be the frame line P. For example, as illustrated in FIG. 8 and FIG. 9, the index mentioned above may be position specification lines Q, which approximately specify longitudinal and lateral (top, bottom, left, and right) positions of an organ (the tongue, for example). In this case, the position specification lines Q may be solid lines as illustrated in FIG. 8, or may be dotted lines as illustrated in FIG. 9.

In whichever of the case where the frame line P is displayed on the display 4 as the index and the case where the position specification lines Q are displayed on the display 4 as the index, the information extractor 14 described above extracts information necessary to diagnose the healthiness of the living body from the image which is acquired through imaging performed by the imager 3 and which is located within a range specified by the index. Here, in the case where the index is the frame line P, the range specified by the index is a range inside the frame line P (the range surrounded by the frame line P), while, in the case where the index is the position specification lines Q, the range is a range inside a closed shape of which an outer shape includes the position specification lines Q. In the examples illustrated in FIG. 8 and FIG. 9, the above-mentioned closed shape is an oblong a contour of which includes the four position specification lines Q.

(Frame Display Magnification)

When the pixel pitch of the display 4 is 100 $\mu$m, the 400 pixels, which need to be arranged on each side, as described above, are to be arranged over a length of 40 mm (100 $\mu$m $\times$ 400 = 40000 $\mu$m). In the preliminary imaging, if 50 extra pixels are added on each of the upper, lower, left, and right sides, then the size of display needs to be 500 pixels on each side, and the length of each side becomes 50 mm (100 $\mu$m $\times$ 500 = 50000 $\mu$m). In this case, the proportion of the size of the frame line P with respect to the size of the display screen is 0.8.

In a case where the number of pixels and the size of the display 4 are insufficient, the display magnification of the frame line P on the display 4 may be adjusted. For example, in a case where the pixel pitch of the display 4 is 100 $\mu$m, and the number of pixels on each side is 250 (the length of each side is 100 $\mu$m $\times$ 250 = 25000 $\mu$m = 25 mm), the display magnification may be $\times$0.5 to display the frame line P in a size such that the number of pixels on each size is 200 (the length of each side is 100 $\mu$m $\times$ 200 = 20000 $\mu$m = 20 mm). In this case, too, the proportion of the size of the frame line P with respect to the size of the display screen is 0.8.

Thus, by displaying the frame line P on the display 4 at a constant proportion relative to the display screen, even when the size (the number of pixels and the pixel pitch) of the display screen varies depending on the display 4 is mounted in the device, it is possible to display the frame line P of a constant size with respect to the display screen. This configuration allows the user to approximately adjust the imaging position of an organ intuitively.

[Specific Method for Extracting Information Necessary for Diagnosis]

As described above, when a captured image of the tongue is acquired by imaging the tongue positioned within the frame line P displayed on the display 4, the above-described information extractor 14 can extract, from the captured image (in particular, the image of the tongue displayed within the frame line), information (such as the color of the tongue) necessary for diagnosis in the following manner. Descriptions will be given below of the method for extracting information on each diagnosis item.

(Extraction of Tongue Color)

FIG. 10 illustrates a captured image of the tongue. In the following description, it is assume that, in the captured image of the tongue, a strip-shaped area of the tongue extending in the top-bottom direction of the tongue in a center in the left-right direction of the tongue is divided into three regions aligned in the top-bottom direction, which are referred to as an upper region R1, a central region R2, and a lower region R3. Here, specific sizes of these three regions are illustrated in FIG. 10, which are determined based on a left-right direction width W and a top-bottom direction length H of an area surrounded by the contour line of the tongue detected by the processor 13, but these are just examples, and not meant as limitation.

The color of the tongue reflects the color of blood, and thus, mainly R and B components vary in RGB image data. Hence, by obtaining R proportion (R/(R+G+B)) or B proportion (B/(R+G+B)) in the lower region R3 of the tongue, the color of the tongue can be extracted in a quantified manner. Here, as the RGB data mentioned above, there can be used the R image data average value, the G image data average value, and the B image data average value of a plurality of pixels in the lower region R3.

The image data of the lower region R3 of the tongue is used to extract the color of the tongue for the following reason. That is, in the tongue diagnosis of the traditional Chinese medicine, the color of the tongue is generally determined by examining right and left end parts or a lower center part of the tongue where there is no tongue coating, but the right and left end parts of the tongue are prone to shadings of color caused by illumination light being incident on the irregularities of the surface of the tongue at various angles, and such shadings of color tend to cause deviation of the image data from values indicating the real color of the tongue.

The information extractor 14 described above may make a judgment on the degree of healthiness by quantifying information extracted from the captured image as necessary. For example, a judgment may be made on the color of the tongue in the following manner. The range of values that R proportion (R/(R+G+B)) can take in the lower region R3 of the tongue is classified into four stages of level 1 to level 4 corresponding to the four colors (reddish purple, pale white, pink, crimson) illustrated in FIG. 5, a judgment is made on which of the level 1 to level 4 the R proportion actually obtained by calculation belongs to, and the judged level is displayed on the display 4. This makes it possible for the user to know the degree of his or her healthiness from the result of the judgment made on the color of his or her tongue (the displayed level). Such a judgment on the level of healthiness can also be made likewise with respect to the following diagnosis items.

(Extraction of Tongue Thickness)

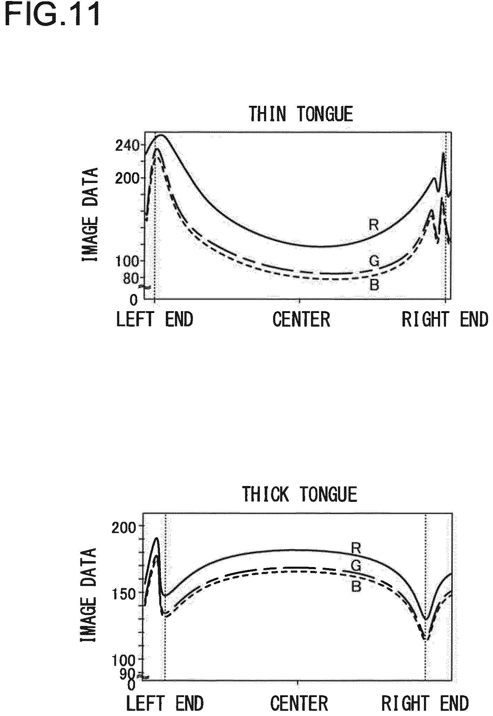

FIG. 11 illustrates the distribution of image data obtained when the surface of the tongue is imaged by the imager 3 under the illumination by the illuminator 2, more specifically, the distribution of RGB image data of a captured image in a horizontal direction passing through substantially the center of the surface of the tongue in the longitudinal direction. The upper graph illustrates the distribution in a thin tongue, while the lower graph illustrates the distribution in a thick tongue. In the graphs, solid lines indicate the distribution of the R image data, alternate long and short dashed lines indicate the distribution of the G image data, and the short dashed lines indicate the distribution of the B image data.

In the case of a thick tongue, the surface of the tongue includes a part that is convex upwardly from its ends toward its center. Since such a convex part of the surface of the tongue is located close to the illuminator 2 and thus is brightly illuminated, the values of the image data increase in the part of the captured image of the tongue corresponding to the convex part. Reversely, in the case of a thin tongue, the surface of the tongue is substantially flat from its ends toward its center, or includes a part that is concave downwardly from its ends toward its center. Such a flat or concave part in the surface of the tongue is located away from the illuminator 2 compared with the convex part described just above, and thus is darker than the convex part when illuminated. Thus, in the captured image of the tongue, in a part corresponding to the flat part or the convex part of the surface of the tongue, the values of the image data are lower than in the part corresponding to the convex part. This tendency applies to all the RGB image data.

To deal with this, the information extractor 14 is configured to be capable of detecting whether the tongue is thick or thin based on irregularities in the horizontal distribution of the image data of any one of RGB (distribution of one color) in the captured image of the tongue acquired under the illumination by the illuminator 2. More specifically, the thickness of the tongue can be accurately detected (regardless of the contour shape of the tongue) by using the horizontal distribution of the image data of any one color of RGB included in the captured image of the tongue as data distribution indicating the degree of irregularities on the surface of the tongue, and quantifying the degree of irregularities into stages of level 1 (the tongue is thick) to level 5 (the tongue is thin). Here, a judgment can be made on the degree of irregularities in the data distribution by, for example, calculating an approximate curve (for example, a quadratic expression) approximating the center part of the data distribution by the least square method or the like, and referring to the coefficient of the quadratic term of the approximate curve and its absolute value. A positive coefficient of the quadratic term of such an approximate curve indicates a concave shape, and a negative coefficient of the quadratic term indicates a convex shape.

The thickness of the tongue can also be detected, with the same accuracy as with this method, by using, as the distribution of the image data, the distribution of data indicating the proportion of the R component (R/(R+G+B)), the proportion of the G component (G/(R+G+B)), and the proportion of the B component (B/(R+G+B)).

(Extraction of Tongue Moisture)

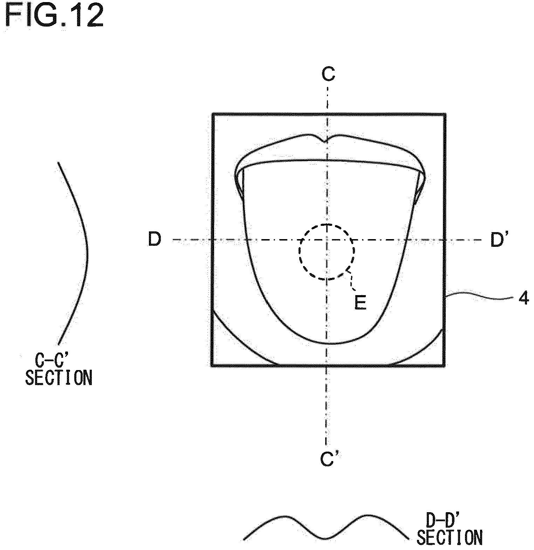

FIG. 12 illustrates a captured image of the tongue along with sectional shapes of the tongue. To be imaged, the tongue is stuck out of the oral cavity. To permit the imager 3 to image the upper lip-side surface of the stuck-out tongue, the surface is curved so as to be convex toward the imager 3 (see the C-C' section).

When the tongue is imaged with the illuminator 2 and the imager 3 arranged as illustrated in FIG. 3, a specular reflection area appears in the upper half of the tongue (because the illuminator 2 is located above the imaging optical axis X). On the other hand, in the left-right direction with respect to the tongue, a middle part of the tongue and the left and right ends of the tongue sag, so that the tongue is curved into an M-shape (see the D-D' section). The tongue has a largely similar sectional shape from an upper to a lower part of it. Moreover, in a central part E of the tongue, a pattern of cracks may be observed. Accordingly, in the present embodiment, an area on the upper half of the tongue excluding a middle part and opposite end parts of it in the left-right direction is set as an area suitable for the detection of the moisture.

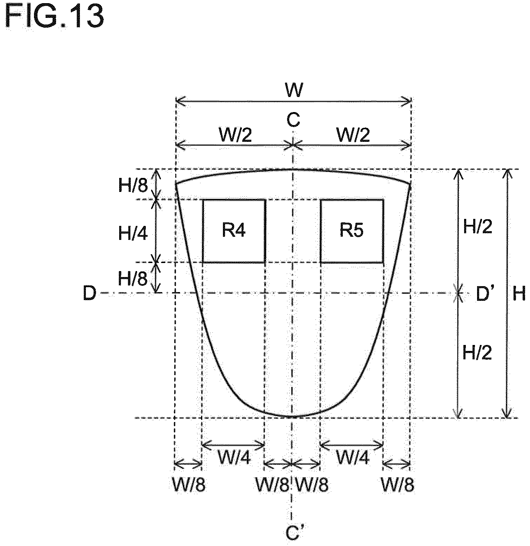

More specifically, from the contour line of the tongue extracted by the processor 13, the information extractor 14 detects the top, bottom, left, and right ends of the tongue to determine the top-bottom length H and the left-right width W of the tongue, and sets moisture detection regions R4 and R5 to be in the mutual positional relationship as illustrated in FIG. 13, with reference to the contour line of the tongue.

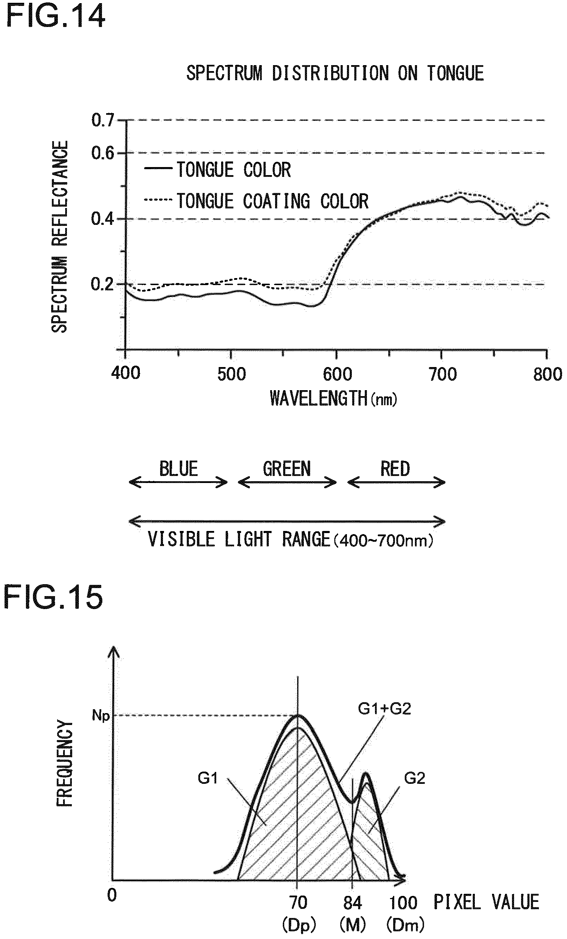

FIG. 14 is a graph illustrating a spectrum distribution on the tongue. The tongue is a mucous membrane without epidermis, and the color of blood appears as the color of the tongue. In the color of blood, the proportion of the R component (with wavelengths from 600 nm to 700 nm) is high, and the proportion of the B component (with wavelengths of 500 nm or less) is low. A lighter color of the tongue indicates a lower proportion of the R component, and a darker color of the tongue indicates a higher proportion of the R component.

On the other hand, the tongue coating is formed of cornified cells of papillae, and takes on a white to yellow color. With a thin tongue coating, the color of the tongue beneath the tongue coating appears, and thus the proportion of the R component is high, as shown in the figure. With a white, thick tongue coating, the proportion of the G component (with wavelengths from 500 nm to 600 nm) is high.

While the colors of the tongue and the tongue coating vary as described above with the health condition of the living body and differences among individuals, the proportion of the B component varies only to a small extent. Thus, in the present embodiment, the degree of gloss on the surface of the tongue is detected, based on the B image data obtained from a captured image of the tongue, through a procedure as described below.

First, the information extractor 14 extracts B image data from pixels in the detection regions R4 and R5 of the captured image, and creates a frequency distribution of the B image data. FIG. 15 schematically illustrates a frequency distribution of the extracted B image data. In FIG. 15, the horizontal axis represents the B pixel value (image data), and the vertical axis represents the frequency (the number of pixels). Here, however, for simple description, it is assumed that a pixel value takes a value in the range from 1 to 100, a greater pixel value indicating a higher brightness.

Next, from the above-mentioned frequency distribution, the information extractor 14 determines the pixel value Dp that corresponds to the highest frequency Np (in the example in FIG. 15, Dp = 70). The information extractor 14 then multiplies that pixel value Dp by 1.2 to determine a threshold value M (in the example in FIG. 15, M = 84). Then, the information extractor 14 adds up the frequencies across the range from the threshold value M to the maximum value of the image data (the maximum pixel value Dm = 100) to obtain the sum of the frequencies as the number of high-value pixels. Here, the pixel value Dp may instead be determined by first finding a function that continuously represents how the frequency varies, then smoothing and removing noise from the function, and then determining the pixel value Dp corresponding to the highest frequency Np. The number of high-value pixels may be determined by integrating the smoothed function across a predetermined range.

Here, if no specular reflection occurs on the tongue surface during imaging, the frequency distribution of the B image data is composed of a single distribution close to a normal distribution (a first group G1). Conversely, if specular reflection occurs, the first group G1 is accompanied by another distribution (a second group G2) with high frequencies at higher pixel values. In addition, since the B image data varies little with the health condition of the living body and differences among individuals as mentioned above, the width of the first group G1 (the width from the minimum to the maximum pixel value in the first group G1) is smaller than the frequency distribution (normal distribution) of other image data, such as R or G image data. As a result, a clear border between the first and the second groups G1 and G2 (a point at which the frequency is minimum and turns from decrease to increase) appears between the value of image data at which the frequency is the highest (i.e., the pixel value Dp) and the maximum value of image data (i.e., the maximum pixel value Dm). This makes it possible to easily distinguish the first group G1 and the second group G2 from each other. In detecting the moisture on the tongue, it is preferable to do so not based on the first group G1, which includes no gloss component (specular reflection component), but based on the second group G2, which represents the gloss component.

Accordingly, the information extractor 14 sets the threshold value M greater than the pixel value Dp, and determines the sum of the frequencies between the threshold value M and the pixel value Dm as the number of high-value pixels, to thereby obtain a value close to the sum of the frequencies in the second group G2.

In particular, it has been experimentally found out that, in the frequency distribution of B image data, the boundary between the first group G1 and the second group G2 appears in the range from 1.1 to 1.3 times the pixel value Dp. Hence, in the present embodiment, the information extractor 14 sets the threshold value M within the range from 1.1 to 1.3 times the pixel value Dp (in the example in FIG. 15, 1.2Dp = 84), and determines the sum of the frequencies between the threshold value M and the pixel value Dm as the number of high-value pixels.

It has been found out that the number of high-value pixels is small in a case where the tongue is dry (the degree of gloss is low), and that the number of high-value pixels is large in a case where the tongue is moist (the degree of gloss is high). Thus, based on the number of high-value pixels, the information extractor 14 can detect the moisture on the tongue by, for example, quantifying the moisture into levels of level 1 (high degree of gloss) to level 3 (low degree of gloss). Further, the information extractor 14 can also make a judgment on the degree of healthiness (normal/abnormal) based on the level obtained by the quantification.

(Extraction of Tooth Mark)

The information extractor 14 approximates the contour line of the tongue obtained by the processor 13 by an approximate curve, and based on the degree of correlation between the contour line and the approximate curve, irregularities in (the smoothness of) the contour line are detected, and thereby a tooth mark on the tongue is detected. The approximate curve is a smooth curve with no fine irregularities, and thus it can be said that the closer the contour line is to the approximate curve, the smoother the contour line is, and the smaller number of tooth marks the tongue has. That is, the higher the degree of correlation between the contour line and the approximate curve is, the smaller number of tooth marks the tongue has; the lower the degree of correlation is, the larger number of tooth marks the tongue has.

Accordingly, the state of a tooth mark can be detected based on the degree of correlation. For example, if the degree of correlation is high, it can be judged that the tooth mark is in a mild state (level 1), and if the degree of correlation is low, it can be judged that the tooth mark is in a severe state (level 3), and if the degree of correlation is between these two, it can be judged that the tooth mark is in a state between the mild and severe states (level 2).

As indices that indicate the degree of correlation between the two, there can be used the following first to third indices.

A first index that indicates the degree of correlation is a determination coefficient $R^2$ given by the following formula:

$$R^2 = 1 - \{(\Sigma(yi - fi)^2)/(\Sigma(yi - Y)^2)\}$$

where

i represents a value between j and k, with j and k representing the x coordinates of one and the other ends, respectively, of the contour line or the approximate curve on the xy plane;
yi represents the y coordinate of the point on the contour line at the x coordinate i on the xy plane;
fi represents the y coordinate of the point on the approximate curve at the x coordinate i on the xy plane; and
Y represents the average value of yi of all points on the contour line. Here, i, j, and k are all integers, fulfilling $j < k$ and $j \leq i \leq k$. $\Sigma(yi-fi)^2$ represents the sum of $(yi-fi)^2$ as i is varied from j to k, and $\Sigma(yi-Y)^2$ represents the sum of $(yi-Y)^2$ as i is varied from j to k.

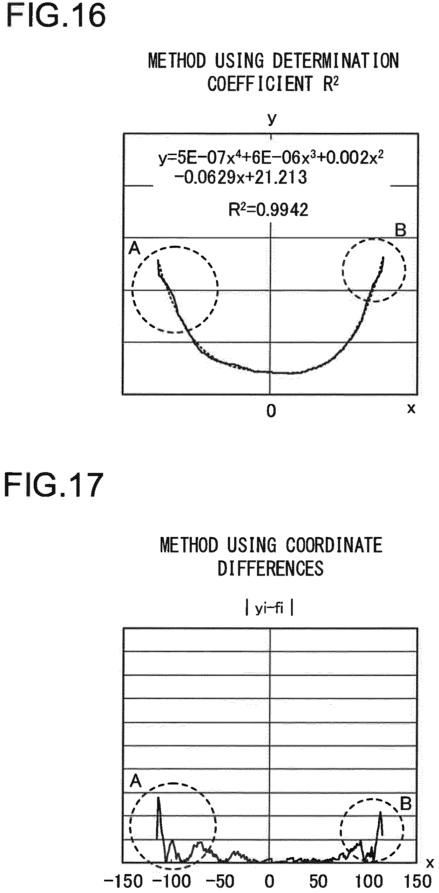

FIG. 16 shows the contour line of the tongue (see the solid line) and an approximate curve (see the dotted line) that approximates it, the polynomial that expresses the approximate curve, and the determination coefficient $R^2$. The approximate curve is found by the least-square method, and is expressed by the polynomial below. Here, the determination coefficient $R^2$ equals 0.9942. In a coefficient in the approximate curve illustrated in the figure, the notation "E - n" stands for $\times 10^{-n}$.

$$y = 5 \times 10^{-7} \cdot x^4 + 6 \times 10^{-6} \cdot x^3 + 2 \times 10^{-3} \cdot x^2 + 6.29 \times 10^{-2} \cdot x + 21.213$$

Both in part A, with a severe tooth mark (large irregularities in the contour line), and in part B, with a mild tooth mark (small irregularities in the contour line), the differences between the contour line and the approximate curve are reflected in the determination coefficient $R^2$. Accordingly, by use of a method that detects a tooth mark based on the determination coefficient $R^2$, even with only a mild tooth mark, it is possible to detect it based on the determination coefficient $R^2$, and this contributes to improved accuracy in tooth-mark detection.

A second index that indicates the degree of correlation is a value obtained based on differences in coordinates (y coordinates) between the contour line and the approximate curve, and is, specifically, the maximum value of |yi - fi| (hereinafter also referred to as "maximum value of coordinate differences").

Here,
i represents a value between j and k, with j and k representing the x coordinates of one and the other ends, respectively, of the contour line or the approximate curve on the xy plane;
yi represents the y coordinate of the point on the contour line at the x coordinate i on the xy plane; and
fi represents the y coordinate of the point on the approximate curve at the x coordinate i on the xy plane. Here, i, j, and k are all integers, fulfilling $j < k$ and $j < i \leq k$.

FIG. 17 is a graph obtained by plotting y-coordinate differences (|yi-fi|) between the tongue contour line in FIG. 16 and its approximate curve (xy polynomial). Both in part A, with a severe tooth mark, and in part B, with a mild tooth mark, the value of |yi - fi| is greater than elsewhere, with no tooth mark. Hence, even with only a mild tooth mark, it is possible to detect it, by detecting the maximum value of |yi - fi|, based on this value, and this contributes to more accurate tooth-mark detection.

A third index that indicates the degree of correlation is a value obtained based on differences in coordinates (y coordinates) between the contour line and the approximate curve, and is a coefficient A given by the following formula:

$$A = \Sigma|yi - fi|$$

where

i represents a value between j and k, with j and k representing the x coordinates of one and the other ends, respectively, of the contour line or the approximate curve on the xy plane;
yi represents the y coordinate of the point on the contour line at the x coordinate i on the xy plane; and
fi represents the y coordinate of the point on the approximate curve at the x coordinate i on the xy plane. Here, i, j, and k are all integers, fulfilling $j < k$ and $j \leq i \leq k$. $\Sigma|yi - fi|$ represents the sum of $|yi - fi|$ as i is varied from j to k (hereinafter also referred to as "sum of coordinate differences").

Using the sum of coordinate differences instead of the maximum value of coordinate differences allows tooth-mark detection that accurately reflects the influence of a fine tooth mark, and thus contributes to still more accurate tooth-mark detection.

As the degree of correlation, it is also possible to use the reciprocal of the sum of the coordinate differences, that is, $1/(\Sigma|yi - fi|)$.

In the above description, the information extractor 14 calculates an approximate curve approximating the contour line of the tongue including a tip part (the tip end) of the tongue, and detects irregularities in the contour line (a tooth mark on the tongue) based on the degree of correlation between the contour line and the approximate curve, but the information extractor 14 may calculate an approximate curve approximating a part of the contour line of the tongue excluding the tip part thereof, and detect irregularities in the contour line based on the degree of correlation between the part of the contour line and the approximate curve. The tip part of the tongue can have varying shapes, like a V shape and a W shape, due to causes other than those intended to be diagnosed, such as poor water metabolism. By detecting irregularities in the contour line based on the degree of correlation between a part of the contour line of the tongue excluding the tip part thereof and the approximate curve, it is possible, without being influenced by the shape of the tip part of the tongue (while eliminating the influence of tenseness and differences among individuals), to detect irregularities (a tooth mark) resulting from poor water metabolism, which is a cause intended to be diagnosed. Thus, it is possible to reduce the inconvenience of the degree of correlation falling down under the influence of the shape of the tip part of the tongue and leading to an incorrect diagnosis of more irregularities than there actually are, and thus to make a more accurate diagnosis based on irregularities in the contour line.

(Extraction of Fissure Pattern (Crack))

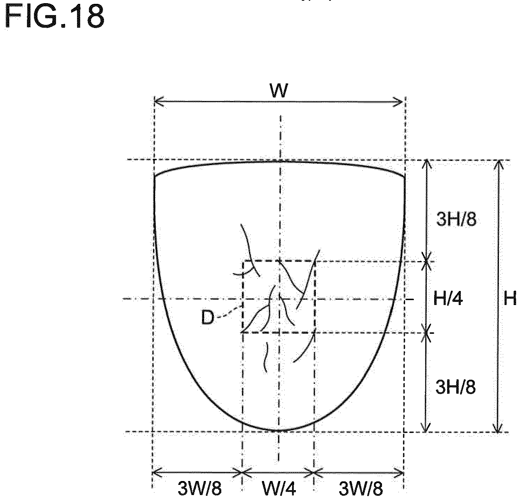

FIG. 18 illustrates a captured image of a tongue having cracks in its surface. To be imaged, the tongue is stuck out of the oral cavity. The imager 3 images the upper lip-side surface of the protracted tongue. Generally, many of cracks in the surface of the tongue are observed in a central area of the tongue, and thus, in the present embodiment, a top-bottom and left-right center part of the tongue (an area including the center of the tongue in the top-bottom and left-right directions) is set as an area suitable for the detection of cracks.

More specifically, from the contour line of the tongue extracted by the processor 13, the information extractor 14 detects the top, bottom, left, and right ends of the tongue to detect the top-bottom length (longitudinal dimension) H and the left-right width (lateral dimension) W of the tongue, and sets, as a crack detection region D of the tongue, a central area of which the longitudinal dimension and the lateral dimension are respectively H/4 and W/4, determined in the dimensional relationship as illustrated in FIG. 18

In a case with some cracks in the surface of the tongue, a base of the tongue appears more than in a case without any cracks in the surface of the tongue, and thus the range of values that the image data of pixels constituting the base of the tongue can take becomes wider with respect to all of RGB. As a result, when a frequency distribution of image data of a captured image is created, the width of the frequency distribution becomes wider. In particular, since the base strongly shows the color of blood, the width of the frequency distribution is significantly wider regarding R and B, which are included in the color of blood by large amounts, as compared with the case without any cracks. It has been found that this tendency is observed regardless of the thickness of the coating on the surface of the tongue or the length of cracks.

Thus, according to the present embodiment, the information extractor 14 creates a frequency distribution of the B image data, for example, from a captured image of the tongue (in particular the image in the detection region D mentioned above), and also, as an index indicating the width of the frequency distribution, calculates and acquires a standard deviation $\sigma$, which indicates the dispersion of the image data. The standard deviation $\sigma$ is the positive square root of dispersion $\sigma^2$ given by the following formula, when the value of the image data takes N values $X_1, X_2, \ldots X_N$.

[Formula 1]

$$\sigma^2 = \frac{1}{N} \sum_{i=1}^{N} (X_i - m)^2$$

where

$$m = \frac{1}{N} \sum_{i=1}^{N} x_i$$

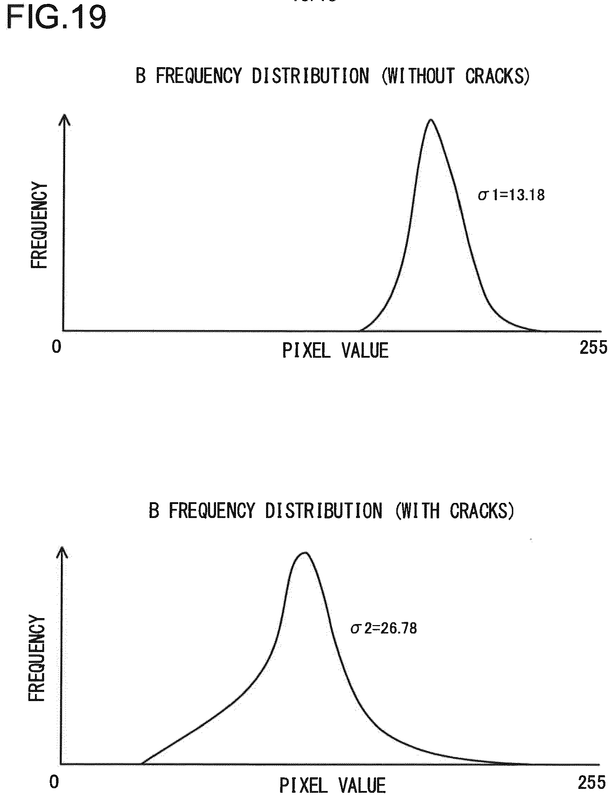

FIG. 19 schematically illustrates the frequency distribution of the B image data created by the information extractor 14; the upper graph illustrating frequency distribution is of a case without any cracks in the tongue surface, while the lower graph illustrating frequency distribution is of a case with some cracks in the tongue surface. In both of them, the horizontal axis represents the B pixel value (image data), and the vertical axis represents the frequency (the number of pixels). The pixel value takes a value in the range of 0 to 255, and the larger the pixel value of a pixel is, the brighter the pixel is.

Standard deviation $\sigma 1$ in the upper graph of frequency distribution was calculated and $\sigma 1 = 13.18$ was obtained. Standard deviation $\sigma 2$ in the lower graph of frequency distribution was calculated and $\sigma 2 = 26.78$ was obtained. This shows that the standard deviation $\sigma$ is larger and the width of the frequency distribution is wider with some cracks in the tongue surface than without any cracks in the tongue surface. Incidentally, an average value m1 of the pixel values in the frequency distribution in the upper graph was calculated and m1 = 177.71 was obtained, and an average value m2 of the pixel values in the frequency distribution in the lower graph was calculated and m2 = 112.75 was obtained.

It has been found out that the standard deviation in the above-described frequency distribution is large in cases with a large number of cracks, and small in cases with a small number of cracks. Accordingly, the information extractor 14 can detect cracks in quantities classified into, for example, level 1 (a large number of cracks) to level 3 (a small number of cracks) based on the standard deviation in the above-described frequency distribution. Here, in the above example, the frequency distribution of the B image data is used to detect cracks, but it is also possible to use other image data, such as of R or G, to detect cracks.

(Extraction of Smoothness of Tongue Coating)

The smoothness of the tongue coating varies depending on the amount of substances adhered to the papillae of the tongue. Specifically, in a case where the amount of such substances is small, the tongue coating is thin enough to allow individual papillae to be recognized one by one, and thus appears to have a rough surface. On the other hand, in a case where the amount of such substances is large, the tongue coating is thick, and since the papillae cover the surface of the tongue, the tongue coating appears to have a flat surface. Thus, the information extractor 14 can detect smoothness of the tongue coating based on the proportion between the number of pixels regarding the papillae and the number of pixels regarding the base.

Specifically, with respect to a region near the center of the tongue where coating adheres (for example, the central region R2 in FIG. 10), the information extractor 14 makes a distinction between pixels of the papillae and those of the base based on a threshold value of a color (R, for example), and calculates the proportion between the number of pixels in the papillae and the number of pixels in the base. When the proportion of the base is high, the coating can be judged to be thin and have a rough surface, and when the proportion of the papillae is high, the coating can be judged to be thick and have a flat surface. By associating the range of the proportion of the number of pixels with the three stages of smoothness of the tongue coating illustrated in FIG. 5, it is possible to judge the degree of smoothness of the tongue coating based on the proportion of the number of pixels actually obtained by calculation.

(Control Flow)

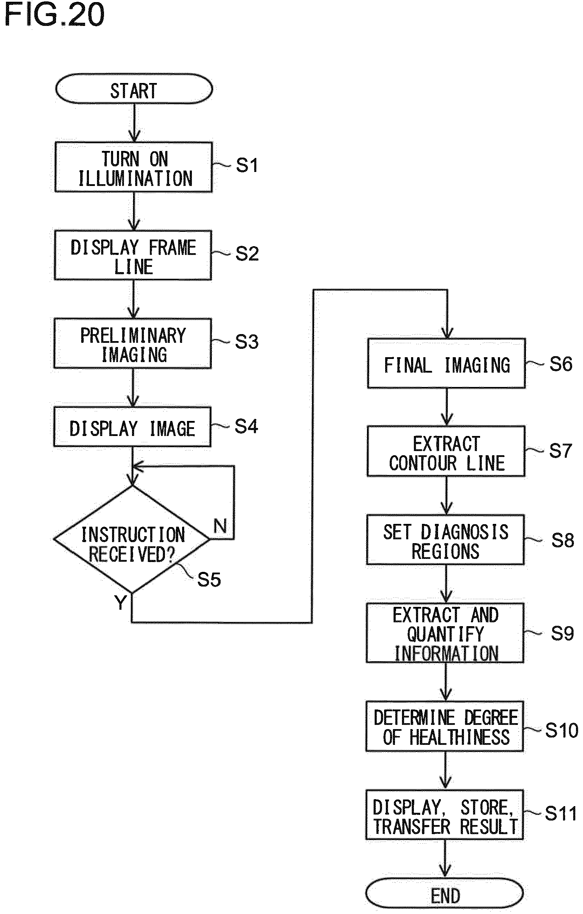

FIG. 20 is a flow chart illustrating the flow of operation in the organ image capture device 1 according to the present embodiment. On receiving an imaging instruction given by pressing an OK button 5a of the operation unit 5, the illumination controller 9 turns on the illuminator 2 (S1), and the display controller 10 makes the display 4 display the frame line P, which specifies the imaging position where required values can be obtained as the illumination angle, the illuminance, and the resolution (S2). Then the imager 4 starts preliminary imaging (S3), and the display 4 displays in real time a video image (preview image) of the tongue of the user along with the frame line P (S4). Thereby, it is possible to urge the user to check the imaging of the tongue as an imaging object and the imaging composition including the angle of the tongue and the way of sticking out the tongue, and the user can so adjust the imaging position of the tongue as to place the captured image of the tongue inside the frame line P.

When the composition is determined, and an instruction to perform final imaging given by the user via the operation unit 5 is received (S5), the imager 3 performs final imaging of the imaging object, and thereby a captured image of the tongue is acquired (S6). The processor 13 extracts a contour line of the tongue from the captured image obtained through the final imaging (S7). Then, based on the contour line of the tongue extracted by the processor 13, the information extractor 14 sets diagnosis regions for extracting information of the diagnosis items (S8). Here, the diagnosis regions correspond to, for example, the regions (the upper region R1, the central region R2, the lower region R3, the detection regions R4 and R5) illustrated in FIG. 10 and FIG. 13, and the detection region D illustrated in FIG. 18. Needless to say, when the imaging position is adjusted and the captured image of the tongue is placed inside the frame line P on the display 4, these diagnosis regions are also located inside the frame line P on the display screen.

After the diagnosis regions are each set in S8, the information extractor 14 extracts, from images of the diagnosis regions, information necessary for diagnosis (tongue color, tongue thickness, tongue moisture, a tooth mark, a fissure pattern, coating smoothness), and quantifies such information (S9). Specifically, in S9, based on the RGB image data in each of the diagnosis region, by means of the above-described method, information on tongue color, etc. is extracted (detected), and the extracted information on each of the diagnosis items is quantified in such a manner, for example, that the tongue color is "level 3" of the four stages of tongue color and the tongue thickness is "level 1" of the three stages of tongue thickness.

Next, the information extractor 14 makes a judgment on the degree of healthiness of the user based on the quantified information (S10). For example, regarding the tongue color, which has been judged to be "level 3", healthiness is judged to be of a normal degree (the tongue color is pink, indicating good health with a good blood flow), and regarding the tongue thickness, which has been judged to be "level 1", healthiness is judged to be of an abnormal degree (the tongue is thin, indicating poor blood flow and water deficiency).

The information quantified, and the result of judgment on the degree of healthiness made, by the information extractor 14 are displayed on the display 4 and stored in the storage unit 15 (S11). From what is displayed on the display 4, the user is able to know the quantified information regarding the diagnosis items or the degree of healthiness. Here, the processing performed in S10 may be omitted (that is, without the device making a judgment on the degree of healthiness), and instead, in S11, the information quantified by the information extractor 14 may be externally transmitted for a judgment to be made on the degree of healthiness of the user outside the device, and the result of the judgment may be then sent to the device to be displayed on the display 4.

As has been described above, in the present embodiment, for the purpose of specifying the imaging position of an organ (the tongue), the imaging position satisfying the imaging conditions for imaging performed by the imager 3, including a condition regarding illumination, the frame line P is displayed on the display 4 along with an image (a preview image) acquired through imaging performed by the imager 3 (see FIG. 6, and S2 to S4 in FIG. 20). Thereby, the most appropriate imaging conditions are automatically set when the user just adjusts the imaging position of the tongue to place the captured image of the tongue inside the frame line P. Thus, even when the user is not experienced in operating the present device, it is possible for him or her to have the imager 3 capture an image of the tongue constantly in the optimal imaging conditions, regardless of when the imaging is performed or who is the examinee, and thus to easily and stably acquire images most appropriate for the diagnosis of the degree of healthiness.

The imaging conditions include the illumination-angle condition. Here, at the imaging position where the imaging conditions are satisfied, the tongue is illuminated at an appropriate illumination angle, and hence, the imager 3 is allowed to image the thus appropriately illuminated tongue to capture an image most appropriate for the diagnosis of the degree of healthiness.

In particular, in the diagnosis regarding tongue moisture or tooth marks, the illumination angle at the time of imaging is important for detecting illumination light (reflected components of the illumination light) reflected on the tongue surface, and for facilitating the detection of the contour line of the tongue by producing shades around the tongue. Thus, the above-discussed configuration is very effective which is provided with the frame line P for specifying the imaging position that satisfies the illumination-angle condition included in the imaging conditions.

The imaging conditions also include the illuminance condition, regarding the illuminance on the tongue. In this case, at the imaging position satisfying the imaging conditions, an appropriate illuminance can be obtained on the tongue by the illumination provided by the illuminator 2, and this allows the imager 3 to image the tongue the illuminance on which is thus appropriate to acquire an image most appropriate for the diagnosis of the degree of healthiness.

As described above, in particular, in the diagnosis of the tongue color or the tongue thickness, a low illuminance lowers the S/N ratio of the imaging signal, and makes accurate detection and diagnosis difficult. To prevent this inconvenience, it is necessary to secure a constant illuminance. For this purpose, the above-discussed configuration where the frame line P is provided to specify the imaging position where the illuminance condition is satisfied as an imaging condition is very effective.

The imaging conditions further includes the resolution condition regarding the resolution of the captured image. In this case, by imaging the tongue at the imaging position satisfying the imaging conditions, it is possible to acquire an image of a desirable resolution for the diagnosis of the degree of healthiness.

In particular, in the diagnosis of a fissure pattern or of the smoothness of tongue coating, a low image resolution makes it impossible to appropriately detect the state of papillae on the surface of the tongue to make an appropriate diagnosis regarding a fissure pattern of the tongue or the smoothness of the tongue coating. To prevent such inconvenience, the above-discussed configuration is very effective in which the frame line P is provided to specify the imaging position where the image-resolution condition is satisfied as an imaging condition.

Further, the display 4 displays the frame line P along with a preview image. This allows the user to adjust the imaging position of the tongue to an appropriate position that satisfies the imaging conditions, before final imaging. Then, from an image acquired in the final imaging that is located inside the frame line P (image in the diagnosis regions), the information extractor 14 extracts information necessary for diagnosis, and thereby, an appropriate diagnosis of the degree of healthiness can be made by the present device or by an external device.

Here, in the above description, it is assumed that the required illumination angle is 30°, the required illuminance is 5000 lx, and the required image resolution is 400×400 pixels, but these numeral values are merely examples, and may be changed as necessary. Then, the size of the frame line P displayed on the display 4 or its display position on the display 4 may be changed according to the changed numeral values, if necessary.

[Program]

The organ image capture device 1 described above may be constituted by, for example, a multifunction portable terminal (computer) such as a smart phone in which a predetermined program (application software) is installed. The computer (for example, the general controller 20 as a CPU) reads and executes the program, and thereby, the above-described variety of processing can be achieved in the organ image capture device 1. Such a program can be obtained by downloading via a network, for example, to be stored in the storage unit 15.

[Other]

The above description describes that the contour line of the tongue is extracted based on a luminance edge in a captured image (a part of the image where brightness changes sharply), and immediately thereafter, the diagnosis regions are set to extract information therefrom. Here, after the extraction of the contour line of the tongue, a judgment may be automatically made on whether the contour line is along the frame line P. Thereby, it is possible to prevent an error of inadvertently capturing an image that does not satisfy required conditions.

For example, in the flow chart of FIG. 20, after the extraction of the contour line by the processor 13 in S7, the information extractor 14 may detect an amount of deviation of the extracted contour line from the frame line P (amounts of deviation in the upward, downward, leftward, and rightward directions and in a rotation direction), and if the amount of deviation is equal to or greater than a threshold value, an alarm unit may give out an alarm (for example, the display 4 may display an alarm display or the audio output unit 8 may output an alarm sound). By means of such an alarm, it is possible to urge the user to correct the composition (the imaging position). Then, after the correction of the imaging position, it is possible to acquire a necessary image through imaging at the appropriate imaging position (under appropriate imaging conditions). Here, after the alarm is given as described above, the procedure returns to S3 and operations in S3 and steps following S3 are repeated.

Although the above description has dealt with a case where the living body is a human, the living body may be any animal other than a human. For example, also with the tongue of a pet or other domestic animal, the procedure according to the embodiment can be used to extract information regarding diagnosis items and to make a diagnosis based on the extracted information. In this way, it is possible for the user to quickly recognize, and precisely deal with, poor health condition of an animal, which cannot communicate by words. The average size of the tongue varies depending on the species of animals to be imaged, thus conditions of the imaging magnitude, the display magnitude, the illuminance, and the like may be appropriately set to be suitable to each species of animals.

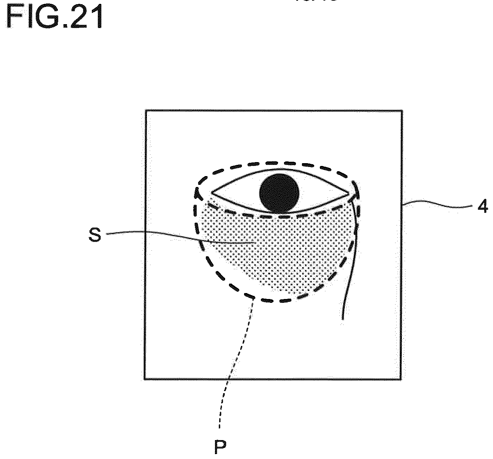

Although the above description has dealt with a case where the imaging object is the tongue of a living body, the imaging object is not limited to the tongue. FIG. 21 illustrates a captured image acquired by imaging a lower eyelid S of a human, which is located under a human eye (the right eye, for example). From the color and shape of the lower eyelid S, it is possible to make a diagnosis on whether blood flow and water metabolism are good or poor. By displaying, on the display 4, the eye and a frame line P as a guide for an area under the eye as illustrated in the figure, it is possible to locate the lower eyelid S at an imaging position where imaging conditions are satisfied, to image the lower eyelid S at the position under appropriate imaging conditions, and to acquire the most appropriate image, based on which to make a diagnosis.

Although the above description deals with a case where the organ image capture device 1 incorporates the illuminator 2, but the illuminator 2 does not necessarily need to be incorporated in the organ image capture device 1. Specifically, the illuminator may be an illuminator separate from the organ image capture device 1, such as a retrofittable light or lamp capable of providing required brightness. In this case, by having the operation unit 5 composed of soft keys (keys for inputting numbers, letters, and the like) on the display 4 (a touch panel display device 41) of the organ image capture device 1, and by the user pressing these soft keys to input a distance (that corresponds to the distance A in FIG. 3) between the imaging optical axis X and an external illuminator, it is possible to achieve imaging at an imaging position satisfying the imaging conditions as much as in the present embodiment. In this case, where the organ image capture device 1 includes no illuminator 2, compactness and cost reduction of the device can be achieved.

As has been discussed above, according to the organ image capture device 1 of the present embodiment, even in cases where the user who is going to image an organ is an inexperienced doctor, a medical professional such as a nurse, a care person, or a pharmacist, or a patient himself/herself, it is possible to always image the organ at an imaging position that satisfies the imaging conditions, and thus to always acquire the most appropriate image, based on which to make a diagnosis. This makes it possible to stably make a diagnosis based on a captured image without variation in imaging conditions depending on the imaging date or the examinee. This further makes it possible to make a comparison between images or results of diagnosis captured or made on different days, or between images or results of diagnosis of different examinees. In particular, combination of imaging performed by a patient himself/herself with computer diagnosis contributes to daily personal health management and early detection of disease.

According to the embodiment described above, an organ image capture device includes an imager which images an organ of a living body to acquire an image of the organ, a display which displays, along with the image acquired through the imaging performed by the imager, an index for specifying an imaging position of the organ where imaging conditions for imaging performed by the imager, including a condition regarding illumination, are satisfied, and an information extractor which extracts information necessary for diagnosis of the degree of healthiness of a living body from an image acquired through the imaging performed by the imager and located inside a range specified by the index.

With the above configuration, the user can appropriately adjust the imaging position of the organ such that a captured image of the organ is located inside the range specified by the index by moving frontward, backward, leftward, rightward, upward, and downward with respect to the imager while watching the index (such as a frame line or a position specification line) and the captured image of the organ displayed on the display. At an imaging position that allows the captured image of the organ to be located inside the range specified by the index, the imaging conditions, including a condition regarding illumination, are satisfied, and thus values required as the angle of illumination and the illuminance, for example, can be obtained. This allows the imager to image the organ under appropriate imaging conditions to acquire an image, allows the information extractor to appropriately acquire information (such as information of the tongue color and the moisture on the tongue) necessary for diagnosis of the degree of healthiness from the captured image of the organ located inside the range specified by the index.

Thus, by the user just adjusting the imaging position of the organ such that the captured image is located inside the range specified by the index, the imaging conditions are automatically set to the most appropriate imaging conditions, and the imager can image the organ under the most appropriate imaging conditions to acquire the most appropriate image based on which to diagnose the degree of healthiness. As a result, even when the user is not experienced in operating the present device, it is possible for him or her to easily and stably acquire an image most appropriate for the diagnosis of the degree of healthiness, regardless of when the imaging is performed or who is the examinee.

The device described above may further include an illuminator which illuminates the organ when the imager images the organ, and the imaging conditions may further include, as a condition regarding illumination, a condition of the illumination angle of the illuminator with respect to the imaging optical axis of the imager. In this case, it is possible for the imager to image an organ illuminated by the illuminator at a required angle to acquire an image most appropriate for the diagnosis of the degree of healthiness.

The device described above may further include an illuminator which illuminates the organ when the imager images the organ, and the imaging conditions may further include, as a condition regarding illumination, a condition of illuminance provided on the organ by the illuminator. In this case, it is possible for the imager to image an organ illuminated by the illuminator with a required illuminance to acquire an image most appropriate for the diagnosis of the degree of healthiness.

The imaging conditions may further include a condition of resolution of an image captured by the imager. In this case, it is possible for the imager to image an organ to acquire an image of a required resolution, that is, an image most appropriate for the diagnosis of the degree of healthiness.

The organ may be a tongue, and the information necessary for the diagnosis may be information regarding moisture or a tooth mark on the tongue. What is important in the diagnosis of the moisture or a tooth mark on the tongue is the angle of illumination applied to the tongue when a captured image from which the information is extracted is acquired (that is, when the tongue is imaged). For this reason, the above-described configuration is very effective in which the imaging conditions include the condition of the illumination angle.

The organ may be a tongue, and the information necessary for the diagnosis may be information regarding the color of the tongue or the thickness of the tongue. What is important in the diagnosis of the color of the tongue or the thickness of the tongue is the illuminance on the tongue when the tongue is illuminated when a captured image from which the information is extracted is acquired (that is, when the tongue is imaged). For this reason, the above-described configuration in which the imaging conditions include the condition of the illuminance on the tongue is very effective.

The organ may be a tongue, and the information necessary for the diagnosis may be information regarding a fissure pattern in the tongue or the smoothness of tongue coating. What is important in the diagnosis of a fissure pattern in the tongue or the smoothness of the tongue coating is the resolution of an image as an imaging condition. For this reason, the above-described configuration is very effective in which the imaging conditions include the image-resolution condition.

The display may display the index in a constant proportion relative to the display screen. Whatever size (the number of pixels and the pixel pitch) the display screen of the display incorporated in the device may have, it is possible to display the index in a constant proportion relative to the display screen.

The display may display the index along with a preview image which is acquired through preliminary imaging performed before final imaging performed by the imager, and the information extractor may extract information necessary for diagnosis from an image which is acquired through the final imaging performed by the imager and which is located inside a range specified by the index. With the preview image and the index displayed on the display, before the final imaging, the user can adjust the imaging position of the organ to an appropriate position that satisfies the imaging conditions. Further, by the information extractor extracting the information necessary for the diagnosis from an image acquired by the final imaging that is located inside the range specified by the index, it is possible for the present device or an external device to appropriately diagnose the degree of healthiness.

The index may be a frame line, and the range specified by the index may be a range surrounded by the frame line. When the index is a frame line, the user can easily recognize the index, and thus can easily adjust the imaging position of the organ such that the captured image of the organ is located inside the frame line.

The index may be a position specification line which specifies longitudinal and lateral positons of the organ, and the range specified by the index may be a range inside a closed shape of which an outer shape includes the position specification line. In this case, too, the user can recognize the index and adjust the imaging position of the organ such that the captured image of the organ is located inside the closed shape mentioned above.

The program according to the present embodiment described above is a program for causing a computer to execute processing of displaying, on a display, an index for specifying an imaging position of an organ of a living body when an imager images the organ, the imaging position of the organ that satisfies imaging conditions including a condition regarding illumination, along with an image acquired through imaging performed by the imager, and processing of extracting information necessary for diagnosing the degree of healthiness of a living body from an image which is acquired through imaging performed by the imager and which is located inside a range specified by the index. By the computer running the program, it is possible to achieve an organ image capture device that performs the above processing.

Industrial Applicability

The present invention is applicable to a device that images an organ of a living body and extracts, from the captured image, information necessary for diagnosis of the degree of healthiness.

List of Reference Signs

1    organ image capture device
2    illuminator
3    imager
4    display
14    information extractor
P    frame line (index)
Q    position specification line (index)

**Claims**

**1.** An organ image capture device comprising:

an imager which images an organ of a living body to acquire an image;
a display which displays, along with the image acquired through imaging performed by the imager, an index for specifying an imaging position of the organ where the organ is to be located when the imager images the organ, the imaging position satisfying imaging conditions including a condition regarding illumination; and
an information extractor which extracts information necessary for diagnosing a degree of healthiness of the living body, from an image which is acquired through imaging performed by the imager and which is located inside a range specified by the index.

2. The organ image capture device according to claim 1, further comprising an illuminator which illuminates the organ when the imager images the organ,
wherein
the imaging conditions include, as the condition regarding illumination, a condition of an illumination angle of the illuminator with respect to an imaging optical axis of the imager.

3. The organ image capture device according to claim 1 or 2, further comprising an illuminator which illuminates the organ when the imager images the organ,
wherein
the imaging conditions include, as the condition regarding illumination, a condition of illuminance provided on the organ by illumination by the illuminator.

4. The organ image capture device according to claim 2 or 3,
wherein
the imaging conditions further include a condition of resolution of an image captured by the imager.

5. The organ image capture device according to claim 2,
wherein
the organ is a tongue, and
the information necessary for diagnosis is information regarding moisture or a tooth mark on the tongue.

6. The organ image capture device according to claim 3,
wherein
the organ is a tongue, and
the information necessary for diagnosis is information regarding color or thickness of the tongue.

7. The organ image capture device according to claim 4,
wherein
the organ is a tongue, and
the information necessary for diagnosis is information regarding a fissure pattern in the tongue or smoothness of tongue coating.

8. The organ image capture device according to any one of claims 1 to 7,
wherein
the display displays the index at a constant proportion with respect to a display screen.

9. The organ image capture device according to any one of claims 1 to 8,
wherein
the display displays the index along with a preview image acquired through preliminary imaging before final imaging performed by the imager, and
the information extractor extracts information necessary for diagnosis from an image which is acquired through the final imaging performed by the imager and which is located inside the range specified by the index.

10. The organ image capture device according to any one of claims 1 to 9,
wherein
the index is a frame line, and
the range specified by the index is a range surrounded by the frame line.

11. The organ image capture device according to any one of claims 1 to 9,
wherein

the index is a position specification line which specifies longitudinal and lateral positions of the organ, and
the range specified by the index is a range inside a closed shape of which an outer shape includes the position specification line.

12. A program for causing a computer to execute
processing of displaying, on a display, along with an image acquired through imaging performed by the imager, an index for specifying an imaging position of an organ of a living body where the organ is to be located when an imager images the organ, the imaging position of the organ satisfying imaging conditions including a condition regarding illumination, and
processing of extracting information necessary for diagnosing a degree of healthiness of the living body from an image which is acquired through imaging performed by the imager and which is located inside a range specified by the index.

FIG.1

## FIG.2

1

| | | |
|---|---|---|
| GENERAL CONTROLLER | ILLUMINATION CONTROLLER 9 | → ILLUMINATOR 2 |
| 20 | IMAGING CONTROLLER 10 | → IMAGER 3 |
| | DISPLAY CONTROLLER 11 | → DISPLAY 4 |
| | OPERATION CONTROLLER 12 | → OPERATION UNIT 5 |
| | PROCESSOR 13 | |
| | INFORMATION EXTRACTOR 14 | |
| | STORAGE UNIT 15 | |
| | COMMUNICATION CONTROLLER 16 | → COMMUNICATOR 7 |
| | AUDIO OUTPUT CONTROLLER 17 | → AUDIO OUTPUT UNIT 8 |

## FIG.3

# FIG.4

CAPTURED IMAGE

4

EDGE EXTRACTION FILTER

| | −1 | |
|---|---|---|
| −1 | 4 | −1 |
| | −1 | |

EXTRACTED CONTOUR
LINE

# FIG.5

| KAMPO DOCTORS' DIAGNOSIS ITEMS AND NUMBER OF STAGES | | | | |
|---|---|---|---|---|
| OBJECT | ITEM | DETAILS | NUMBER OF STAGES | STATE VARIATION |
| TONGUE | COLOR | BASE COLOR | 4 | REDDISH PURPLE - PALE WHITE -(PINK)- CRIMSON |
| | THICKNESS | THICKNESS OF TONGUE | 3 | THIN -(MODERATE)- THICK |
| | MOISTURE | SURFACE GLOSS | 3 | NONE -(MODERATE)- A LOT |
| | SHAPE (TOOTH MARK) | PERIPHERAL IRREGULARITIES | 4 | (NONE)- FEW - INTERMEDIATE - A LOT |
| | FISSURE PATTERN(CRACKS) | CENTRAL DARK LINE | 4 | (NONE)- FEW - INTERMEDIATE - A LOT |
| TONGUE COATING | COLOR | TONGUE COATING COLOR | 4 | (PALE WHITE)- WHITE -YELLOW - BROWN |
| | SMOOTHNESS | DEGREE OF SEPARATION BETWEEN PAPILLAE | 2 | (SMALL)- LARGE |
| | THICKNESS | TONGUE COATING THICKNESS | 3 | THIN -(MODERATE)- THICK |
| | PRESENCE/ ABSENCE | PRESENCE/ABSENCE OF TONGUE COATING | 2 | ABSENT -(PRESENT) |
| | DISTRIBUTION | UNEVEN DISTRIBUTION OF TONGUE COATING | 2 | (ABSENT)- PRESENT |

⬭ : NORMAL (HEALTHY) STATE

# FIG.6

# FIG.7

5(5a)       5(5b)

| OK | CANCEL |

22

41

4

P

# FIG.8

FIG.9

FIG.10

# FIG.11

THIN TONGUE

THICK TONGUE

# FIG.12

C

D ----------- D'

4

E

C'

C-C'
SECTION

D-D'
SECTION

## FIG.13

## FIG.14

### SPECTRUM DISTRIBUTION ON TONGUE

BLUE ← → GREEN ← → RED ← →

VISIBLE LIGHT RANGE (400~700nm)

## FIG.15

# FIG.16

METHOD USING DETERMINATION
COEFFICIENT $R^2$

$y=5E-07x^4+6E-06x^3+0.002x^2-0.0629x+21.213$

$R^2=0.9942$

# FIG.17

METHOD USING COORDINATE
DIFFERENCES

$|yi-fi|$

# FIG.18

# FIG.19

B FREQUENCY DISTRIBUTION (WITHOUT CRACKS)

$\sigma 1 = 13.18$

B FREQUENCY DISTRIBUTION (WITH CRACKS)

$\sigma 2 = 26.78$

# FIG.20

```
        ┌─────────────┐
        │    START    │
        └─────────────┘
               │
               ▼
        ┌─────────────┐
        │   TURN ON   │──S1
        │ ILLUMINATION│
        └─────────────┘
               │
               ▼
        ┌─────────────┐
        │DISPLAY FRAME│──S2
        │    LINE     │
        └─────────────┘
               │
               ▼
        ┌─────────────┐
        │ PRELIMINARY │──S3
        │   IMAGING   │
        └─────────────┘
               │
               ▼
        ┌─────────────┐
        │DISPLAY IMAGE│──S4
        └─────────────┘
               │
               ▼
            ◇───────
          ◇           ◇   N
        ◇ INSTRUCTION   ◇────
          ◇ RECEIVED? ◇
            ◇───────◇  S5
              Y
```

```
        ┌─────────────┐
        │FINAL IMAGING│──S6
        └─────────────┘
               │
               ▼
        ┌─────────────┐
        │   EXTRACT   │──S7
        │ CONTOUR LINE│
        └─────────────┘
               │
               ▼
        ┌─────────────┐
        │SET DIAGNOSIS│──S8
        │   REGIONS   │
        └─────────────┘
               │
               ▼
        ┌─────────────┐
        │ EXTRACT AND │
        │   QUANTIFY  │──S9
        │ INFORMATION │
        └─────────────┘
               │
               ▼
        ┌─────────────┐
        │DETERMINE DEGREE│──S10
        │OF HEALTHINESS│
        └─────────────┘
               │
               ▼
        ┌─────────────┐
        │DISPLAY, STORE,│──S11
        │TRANSFER RESULT│
        └─────────────┘
               │
               ▼
        ┌─────────────┐
        │     END     │
        └─────────────┘
```

# FIG.21

## INTERNATIONAL SEARCH REPORT

| International application No. |
| --- |
| PCT/JP2015/079123 |

A. CLASSIFICATION OF SUBJECT MATTER

*A61B5/00*(2006.01)i, *A61B5/107*(2006.01)i, *H04N5/225*(2006.01)i

According to International Patent Classification (IPC) or to both national classification and IPC

B. FIELDS SEARCHED

Minimum documentation searched (classification system followed by classification symbols)
A61B5/00-5/053, 5/06-5/22, H04N5/225

Documentation searched other than minimum documentation to the extent that such documents are included in the fields searched

| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| Y | JP 2007-152084 A (Shiseido Co., Ltd.), 21 June 2007 (21.06.2007), paragraphs [0118] to [0123]; fig. 11 & CN 1961820 A | 1-12 |
| Y | JP 2007-141018 A (Fujifilm Corp.), 07 June 2007 (07.06.2007), paragraphs [0027] to [0028]; fig. 3 (Family: none) | 1-12 |
| Y | JP 2005-137756 A (Konica Minolta Holdings, Inc.), 02 June 2005 (02.06.2005), claim 3 (Family: none) | 5-7 |

| ☒ Further documents are listed in the continuation of Box C. | ☐ See patent family annex. |

| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 21 December 2015 (21.12.15) | 28 December 2015 (28.12.15) |

| Name and mailing address of the ISA/ Japan Patent Office 3-4-3,Kasumigaseki,Chiyoda-ku, Tokyo 100-8915,Japan | Authorized officer |
| --- | --- |
| | Telephone No. |

Form PCT/ISA/210 (second sheet) (July 2009)

**INTERNATIONAL SEARCH REPORT**

International application No.

PCT/JP2015/079123

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-172181 A  (Seiko Epson Corp.), 06 August 2009 (06.08.2009), claims 1 to 4; paragraph [0022]; fig. 4 (Family: none) | 1-12 |
| A | JP 2004-48812 A  (RIC Co., Ltd.), 12 February 2004 (12.02.2004), claim 1; fig. 1 & US 2004/0028263 A1 claim 1; fig. 1 & DE 10309873 A1 | 1-12 |
| P,X | WO 2015/037316 A1  (Konica Minolta, Inc.), 19 March 2015 (19.03.2015), paragraphs [0030] to [0031], [0034] to [0038]; fig. 3, 6 to 7 (Family: none) | 1-12 |
| P,X | JP 2014-219781 A  (NTT Communications Corp.), 20 November 2014 (20.11.2014), claim 8; paragraphs [0034] to [0035]; fig. 3 (Family: none) | 1-4,8-12 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005152569 A
- JP 2002229106 A
- JP H075530 B
- JP 2007166250 A

- JP 2011135527 A
- JP 2009033544 A
- JP 2004040724 A